# EUROPÄISCHE PATENTSCHRIFT

(11) **EP 3 529 620 B1**
(45) Veröffentlichungstag und Bekanntmachung des Hinweises auf die Patenterteilung: **18.11.2020**
(21) Anmeldenummer: 17794240.6
(22) Anmeldetag: 17.10.2017
(51) Int. Cl.: G01N 33/569

(54) **VERFAHREN ZUR IMMUNOLOGISCHEN DIAGNOSE EINER PROBE MIT EINER POTENTIELLEN INFEKTION EINES ARBOVIRUS UND HIERFÜR GEEIGNETE TESTKITS**
VERFAHREN ZUR IMMUNOLOGISCHEN DIAGNOSE EINER PROBE MIT EINER POTENTIELLEN INFEKTION EINES ARBOVIRUS UND HIERFÜR GEEIGNETE TESTKITS
VERFAHREN ZUR IMMUNOLOGISCHEN DIAGNOSE EINER PROBE MIT EINER POTENTIELLEN INFEKTION EINES ARBOVIRUS UND HIERFÜR GEEIGNETE TESTKITS

(30) Priorität: 19.10.2016 EP 16194496
(43) Veröffentlichungstag der Anmeldung: 28.08.2019
(73) Patentinhaber: Mikrogen GmbH, 82061 Neuried (DE)
(72) Erfinder: SOUTSCHEK, Erwin, 82335 Berg (DE); BÖCHER, Oliver, 82061 Neuried (DE); NÖLTING, Christina, 80686 München (DE)
(74) Vertreter: Keller, Günter
(86) Internationale Anmeldenummer: PCT/EP2017/076411
(87) Internationale Veröffentlichungsnummer: WO 2018/073208

(56) Entgegenhaltungen:
- WO-A1-2016/022071
- WO-A1-2016/069245
- US-A1- 2004 197 769
- GINIER MYL GBP ENE ET AL: "Zika without symptoms in returning travellers: What are the implications?", TRAVEL MEDICINE AND INFECTIOUS DISEASE, ELSEVIER, AMSTERDAM, NL, Bd. 14, Nr. 1, 5. Februar 2016 (2016-02-05), Seiten 16-20, XP029432047, ISSN: 1477-8939, DOI: 10.1016/J.TMAID.2016.01.012
- CAROD-ARTAL FRANCISCO J: "Epidemiology and neurological complications of infection by the Zika virus: a new emerging neurotropic virus", REVISTA DE NEUROLOGIA, VIGUERA EDITORES, ES , Bd. 62, Nr. 7 1. April 2016 (2016-04-01), Seiten 317-328, XP008183930, ISSN: 1576-6578 Gefunden im Internet: URL:http://www.neurologia.com/articulo/201 6152/eng [gefunden am 2017-03-28]
- Anonymous: "ZIKA/DENGUE/CHIKUNGUNYA COMBOS", , 2. Juli 2016 (2016-07-02), XP055359891, Gefunden im Internet: URL:http://wayback.archive.org/web/2016070 2052721/http://www.rapidtest.ca/products-d etail.php?id=154 [gefunden am 2017-03-29]
- Anonymous: "Zika virus infections", , 1. Juli 2016 (2016-07-01), Seiten 1-8, XP055359072, Gefunden im Internet: URL:https://www.euroimmun.com/documents/In dications/Infections/Zika-virus/HI_2668_I_ UK_B.pdf [gefunden am 2017-03-27]
- CHUN-WAN YEN ET AL: "Multicolored silver nanoparticles for multiplexed disease diagnostics: distinguishing dengue, yellow fever, and Ebola viruses", LAB ON A CHIP, Bd. 15, Nr. 7, 1. Januar 2015 (2015-01-01) , Seiten 1638-1641, XP055359648, ISSN: 1473-0197, DOI: 10.1039/C5LC00055F
- ALISON J. BASILE ET AL: "Multiplex Microsphere Immunoassays for the Detection of IgM and IgG to Arboviral Diseases", PLOS ONE, Bd. 8, Nr. 9, 25. September 2013 (2013-09-25), Seite e75670, XP055359604, DOI: 10.1371/journal.pone.0075670
- Anonymous: "Zika Virus Envelop (Env) and NS1 Protein ELISA Kits", , 14. Juli 2016 (2016-07-14), Seiten 1-2, XP055359652, Gefunden im Internet: URL:https://www.4adi.com/objects/catalog/p roduct/extras/Zika-Env-NS1-Antigen-ELISA-F lr.pdf [gefunden am 2017-03-28]
- PEI-YUN SHU ET AL: "Current Status of Dengue Diagnosis at the Center for Disease Control, Taiwan Introduction", DENGUE BULLETIN, Bd. 28, 1. Januar 2004 (2004-01-01), Seiten 107-117, XP055359351,

## Beschreibung

Gegenstand der vorliegenden Erfindung ist ein Verfahren zur immunologischen Differenzialdiagnose einer Probe eines Patienten mit einer potentiellen Infektion eines Arbovirus und hierfür geeignete Test-Kits.

Viren, die durch Insekten (vor allem Mücken) oder Spinnentiere (beispielsweise Zecken) übertragen werden, bezeichnet man als Arboviren (arthropod-borne viruses). Voraussetzung für die Übertragung von Viren durch Arthropoden ist, dass die Viren bestimmte Organe der Tiere (vor allem Stechmücken), wie etwa die Epithelzellen des Darms oder der Speicheldrüsen, infizieren können. Grundsätzlich genügt die alleinige Aufnahme von virushaltigem Blut durch Arthropoden nicht. Vielmehr müssen die Viren sowohl in Arthropoden, wie auch in Säugetierzellen einen produktiven Infektionszyklus durchlaufen können.

Durch den in den letzten Jahren äußerst stark zugenommenen Reiseverkehr und den globalen Handel sind sowohl Viren, wie auch Mücken in Gegenden verbracht worden, in denen sie ursprünglich nicht endemisch waren. Dadurch können Erkrankungen und lokale Epidemien auch an solchen geographischen Orten auftreten, an denen sie vorher nicht beobachtet wurden. Weiterhin spielt die starke Industrialisierung und die damit verbundene zunehmende Bevölkerungsdichte eine wesentliche Rolle für die Verbreitung von Epidemien.

Ein großer Teil der Bevölkerung reist in geographisch weit entfernte Gegenden und es ist daher häufig diagnostisch schwierig zu bestimmen, mit welchem Virus der Patient infiziert wurde. Darüber hinaus ist es durchaus möglich, dass Infektionen mit mehreren verschiedenen Viren vorliegen und der Patient kann schon vorher eine möglicherweise inapparent verlaufene Virusinfektion durchlaufen haben. Derzeit sind vier verschiedene Serotypen des Dengue-Virus bekannt. Eine einmal durchgemachte Infektion verleiht keinen dauerhaften Immunschutz gegenüber Infektionen mit einem anderen Serotyp des Dengue-Virus. Daher ist es möglich, dass ein und derselbe Patient mehrmals an Denguefieber erkrankt. Ein weiteres Problem sind Kreuzreaktivitäten, die aufgrund der genetischen Verwandtschaft der Viren und der Ähnlichkeiten der Antigene auftreten können. Eine weitere Komplikation der Diagnostik kann durch Impfungen (beispielsweise gegen das Gelbfieber-Virus) hervorgerufen werden.

Cleton et al. (PLOS Neglected Tropical Diseases, März 2015, S. 1-17) weisen auf die Schwierigkeiten einer Differenzialdiagnostik bei Infektionen durch ein Mitglied der Familie Flaviviridae hin. In dem dort beschriebenen Test werden die NS1-Proteine für die immunologische Diagnostik verwendet.

Die US 2004/0197769 offenbart einen diagnostischen Test zum Nachweis des West-Nil-Virus, des japanischen Encephalitis-Virus, des St. Louis-Encephalitis-Virus und des Dengue-Virus. Dabei wird die Probe mit auf einer Festphase aufgebrachten Antigenen, nämlich dem "non-structural protein 5" und dem "envelope protein" in Kontakt gebracht. Neuere Entwicklungen haben jedoch gezeigt, dass Nicht-Strukturprotein 5 (NS5) nicht so spezifisch ist wie angenommen. Alves et al., Clinical and Vaccine Immunology, 2016, Vol. 23, No. 6, pp 460-469 hatten gezeigt, dass das Dengue-Virus NS5-Protein zwischen den vier DENV-Serotypen die höchste Homologie besitzt und aufgrund von hoch konservierten NS5 Epitopen stark kreuzreaktiv reagiert. Dies steht im Gegensatz zu den Aussagen der US 2004/0197769, wonach NS5 hochspezifisch und zur Unterscheidung verschiedener Flaviviren einschließlich der Typisierung von Dengue-Virus 1-4 geeignet sein soll.

Die Patentinhaberin Susan J. Wong weist selbst in ihrer aktuellen Publikation Wong et al., EBioMedicine (2017), http://dx.doi.org/10.2016/j.ebiom.2017.01.008 darauf hin, dass es zielführend wäre, für eine virus-spezifische serologische Diagnose geeignete spezifische Epitope von NS1, NS5 und Envelope Protein zu identifizieren und für diagnostische Zwecke zu etablieren.

Die WO 2016/022071 offenbart einen "point of care test" zum Nachweis von Dengue-Viren. Es wird jedoch kein Testverfahren zur Differenzierung von verschiedenen nah verwandten Flaviviren offenbart.

Die WO 2016/069245 offenbart eine Vorrichtung zur Detektion von Erregern von verschiedenen fiebrigen Erkrankungen Eine Differenzierung von verschiedenen nah verwandten Erregern ist jedoch weder beabsichtigt noch offenbart.

Gineer et al., Travel Medicine and Infectious Disease (2016), Vol. 14, pp. 16-20 bestätigen, dass die serologische Differenzierung zwischen einer Dengue-Virus- und einer Zika-Virusinfektion aufgrund der hohen Kreuzreaktivität schwierig ist. Auch Carod-Artal, Rev. Neurol., 2016, 62 (7): 317-328 bestätigt die diagnostischen Schwierigkeiten, die auftreten, wenn eine Infektion mit dem Zika-Virus abgegrenzt werden soll von anderen Flavivirusinfektionen wie Dengue, Gelbfieber oder West-Nil-Fieber sowie von einer Chikungunya-Virus Infektion.

Carod-Artal, Rev Neurol 2016, 62 (7). pp 317-328 beschreibt die Epidemiologie und neurologische Komplikationen von Infektionen mit dem Zika-Virus.

Von Biocan Diagnostics Inc. wurde im März 2017 im Internet publiziert, dass ein Schnelltest für den gleichzeitigen Nachweis von IgG- und IgM-Antikörpern gegen Dengue-Virus und Zika-Virus geplant ist.

Im Hinblick auf die Ähnlichkeit der Symptome der viralen Infektionen, sowie der möglichen Mehrfachinfektionen besteht daher ein erhebliches Interesse an einfach durchzuführenden, zuverlässigen differenzialdiagnostischen Verfahren, die den besonderen Schwierigkeiten, insbesondere dem Problem der Kreuzreaktionen Rechnung tragen.

Erfindungsgemäß bevorzugt ist eine immunologische Differenzialdiagnose zur Unterscheidung einer Infektion durch Dengue-Virus, Zika-Virus sowie Chikungunya-Virus. Weiterhin kann auch der Nachweis einer Gelbfieber-Infektion oder -Impfung gezeigt werden, sowie die differentialdiagnostische Unterscheidung.

Bei dem Dengue-Virus handelt es sich um ein Virus mit einzelsträngigem RNA-Genom in Plusstrang-Orientierung. Das Dengue-Virus gehört zu dem Genus Flavivirus der Familie Flaviviridae. Zu dieser Familie gehört auch das Gelbfieber-Virus, das West-Nil-Virus, das japanische Encephalitis-Virus, das St. Louis-Encephalititis-Virus und das Frühsommer-Menengitis-Virus, das auch im Voralpenland gehäuft auftreten kann.

Zu dem Genus Flavivirus gehört auch das Zika-Virus. Im Jahr 1947 wurde das Zika-Virus in Uganda entdeckt und es wurden nur sporadische Ausbrüche des Virus beobachtet. In den letzten Jahren jedoch ist es zu einer explosionsartigen Ausbreitung des Virus gekommen, wobei sich die klinischen Symptome von nahezu symptomfrei, über eine milde fiebrige Erkrankung bis zu schweren Fällen mit neurologischen Komplikationen erstrecken können. Mittlerweile geht man davon aus, dass das Zika-Virus auch durch sexuelle Aktivitäten übertragen werden kann. Bei Infektionen während der Schwangerschaft wurden häufig Fälle von Mikrozephalie beobachtet. Eine zuverlässige Differenzialdiagnostik ist daher insbesondere dann wichtig, wenn es sich bei der Patientin um eine schwangere Frau handelt. Weiterhin ist zu erwähnen, dass das Zika-Virus eine besonders hohe genetische Homologie zu den Dengue-Viren besitzt, was in der Regel ein Problem bei deren Differenzierung darstellt.

Ein anderes zu den Arboviren zählendes Virus ist das Chikungunya-Virus, das zu der Familie der Togaviridae, Gattung Alphavirus, gehört. Dengue-, Zika- sowie Chikungunya-Viren werden auf den Menschen durch Stechmücken der Gattung Aedes übertragen und können sehr ähnliche klinische Symptome wie Fieber, Schmerzen in den Gelenken, Kopfschmerzen, Müdigkeit, Schwindel und Erbrechen hervorrufen.

Aufgabe der vorliegenden Erfindung ist es, ein Verfahren zur immunologischen Diagnose einer mutmaßlichen Infektion mit einem oder mehreren Arbovirus bereitzustellen, wobei ermittelt werden soll, mit welchen Viren der Patient / die Patientin infiziert worden ist.

In den ersten Tagen nach Auftreten der Symptome kann der Erreger mittels NukleinsäureNachweis ermittelt werden. Gegen Ende der Akutphase ist dies jedoch i.d.R. nicht mehr möglich. Dann ist nach internationalen Richtlinien (WHO, CDC) ein serologischer Nachweis die Methode der Wahl.

Weiterhin hat ein immunologisches Diagnoseverfahren den Vorteil, dass die Titer von IgM bzw. IgG dazu verwendet werden können, den Verlauf der Erkrankung zu charakterisieren (akute Phase, kürzlich zurückliegend und abgelaufene/weit zurückliegende Infektion). Nach einer Infektion mit einem Arbovirus steigt zunächst der IgM-Titer stark an und fällt dann wieder innerhalb der nächsten Wochen bzw. Monate unter die Nachweisgrenze. Der IgG-Titer steigt leicht zeitverzögert an und bleibt oft für Jahrzehnte nachweisbar. Bei einem erneuten Kontakt mit dem Erreger steigt der IgG-Titer auf ein deutlich höheres Niveau an, wohingegen der IgM-Titer im Vergleich zur Primärinfektion deutlich weniger ansteigt und sogar unter der Nachweisgrenze liegen kann.

Gegenstand der vorliegenden Erfindung ist ein Verfahren zur immunologischen Differenzialdiagnostik, einer Probe eines Patienten / einer Patientin auf eine potentielle Infektion mit einem Arbovirus, wie in den Patentansprüchen näher definiert.

Bei dem Verfahren wird zunächst die Probe des Patienten in Kontakt gebracht mit mehreren Antigenen, die räumlich getrennt voneinander auf einer Festphase aufgebracht wurden. Die Probe ist in bevorzugter Ausführungsform eine Antikörper enthaltende Probe, also eine Serum- und/oder Plasmaprobe. Andere Körperflüssigkeiten eines Patienten, wie beispielsweise Liquor oder Muttermilch, können in dem Verfahren ebenfalls eingesetzt werden.

Bei den Antigenen werden mehrere unterschiedliche Proteine oder Peptidfragmente mit wenigstens 8, bevorzugt 12 und ganz bevorzugt 40 Aminosäuren eingesetzt. Bei den erfindungsgemäßen Verfahren bzw. den Kits zur Durchführung dieser Verfahren werden die nachfolgend näher aufgeführten Antigene eingesetzt. Es ist möglich, die vollständigen Proteine einzusetzen, oder auch Fragmente dieser Proteine mit den immunologisch reaktiven Teilen. Da die Sequenzen der Antigene bekannt sind, können die Proteine bzw. Proteinfragmente in an sich bekannter Art und Weise rekombinant hergestellt werden. Um herauszufinden, welche Teile immunologisch reaktiv sind, werden üblicherweise diejenigen Teile exprimiert, die sich bei den Antigenen an Stellen befinden, die dem Immunsystem präsentiert werden. Welche Teile besonders geeignet sind, kann leicht dadurch ermittelt werden, dass die exponierten Fragmente rekombinant hergestellt werden und mit bekannten Patientenseren getestet werden. Diese bekannten, wohl definierten Patientenseren stammen von solchen Patienten, bei denen die Infektion mit einem bestimmten Virus über die verschiedenen Diagnosemöglichkeiten sicher nachgewiesen wurde.

Wenn Fragmente eingesetzt werden, weisen diese wenigstens 8, in der Regel wenigstens 40, bevorzugt wenigstens 60 und besonders bevorzugt wenigstens 80 Aminosäuren auf. Besonders bevorzugt sind auch solche Antigene, die dem Volllängenprotein entsprechen oder wenigstens 80 %, bevorzugt wenigstens 90 % und ganz bevorzugt wenigstens 95 % des Volllängenantigens beinhalten. Erfindungsgemäß bevorzugt eingesetzt werden daher Proteine, die zu 80 % und besonders bevorzugt zu 90 % und ganz besonders bevorzugt zu 95 % identisch sind zu den Volllängenproteinen der NS1- und E-Proteine. Eine besonders hohe Identität ist bei den E-Proteinen in den Domänen I-III bevorzugt. In bevorzugter Ausführungsform werden entweder am N-Terminus oder am C-Terminus oder an beiden Enden Fragmente von wenigstens 10, bevorzugt wenigstens 20 Aminosäuren nicht exprimiert.

Es handelt sich hierbei bevorzugt um folgende Antigene:

### a) "non-structural protein 1" (NS1 Antigen) des Dengue-Virus

Es gibt vier Serotypen des Dengue-Virus mit den Bezeichnungen DENV-1, DENV-2, DENV-3 und DENV-4. Erfindungsgemäß wird das NS1 des Serotyps 2 (DENV-2) eingesetzt. Das NS1 ist ein hochkonserviertes Glykoprotein, das scheinbar für die Überlebensfähigkeit des Virus essentiell ist, aber es wurde diesem Antigen noch keine eindeutige biologische Aktivität zugeordnet. Eigentlich unüblich für ein virales Glykoprotein wird NS1 sowohl in Membran-assoziierter, wie auch in sekretierter Form produziert. Daher ist das NS1 Antigen während der frühen klinischen Phase der Erkrankung in hohen Konzentrationen im Serum von mit Dengue-Virus infizierten Patienten vorhanden. Die kompletten Genom-Sequenzen der Dengue-Virus-Typen 1-4 wurden von Añez et al., Genome Announcements (2016), Vol. 4, No. 1, S. 1-2 veröffentlicht.

### b) "envelope protein" des Dengue-Virus

Ein weiteres, bei dem erfindungsgemäßen Testverfahren eingesetztes Protein ist das "envelope protein" des Dengue-Virus Serotyp 2. Das envelope/Hüllprotein E stellt das Hauptantigen für die humorale Immunantwort dar. Hierin auftretende Mutationen können die Virulenz des Dengue-Virus beeinflussen.

### c) "envelope protein 1" des Chikungunya-Virus

Ein weiteres, in dem Test eingesetztes Antigen ist das "envelope protein 1" des Chikungunya-Virus oder ein immunologisch reaktives Teil hiervon mit wenigstens 40 Aminosäuren. Zwischenzeitlich sind drei phylogenetisch unterscheidbare Gruppen des Chikungunya-Virus bekannt, nämlich der asiatische Genotyp, der westafrikanische Genotyp und der ost-, zentral- und südafrikanische Genotyp. Das Genom des Chikungunya-Virus ist ein positivsträngiges RNA-Genom mit etwa 12000 Nukleotiden. Es enthält zwei offene Leserahmen, wobei der zweite Leserahmen für die Strukturproteine C, E1, E2 und E3 kodiert. Eine vollständige Genomsequenz des Chikungunya-Virus mit der Bezeichnung GD05/2010 ist in der Genbank unter der Zugangsnummer JX088705 erhältlich (Li et al., Journal of Virology (2012), Vol. 86, No. 16, S. 8904-8905).

Durch den Einsatz dieses E1-Proteins kann ermittelt werden, ob eine Infektion mit einem Chikungunya-Virus vorliegt. Alternativ können auch "virus-like particles" (VLP) von Chikungunya eingesetzt werden. Solche VLPs können beispielsweise in Insektenzellen hergestellt und auch für Impfungen verwendet werden ([DeZure et al., JID 2016: 214 (Suppl. 5)])

### d) NS1-Protein des Zika-Virus

Ein weiteres, in dem Test eingesetztes Antigen ist das "non-structural protein 1" (NS1) des Zika-Virus oder ein immunologisch reaktives Teil hiervon mit wenigstens 40 Aminosäuren. Das Genom des Zika-Virus besteht aus einer ca. 10000 Nukleotide langen RNA, die ein Polyprotein kodiert, das alle strukturellen Proteine aufweist. Die Genomorganisation umfasst die Proteine C, prM, E, NS1, NS2a, NS2b, NS3, NS4a, NS4b und NS5.

### e) "envelope protein" des Zika-Virus

Das "envelope protein" wird abgekürzt mit "E". Eine komplette Nukleotidsequenz eines Zika-Virus wurde in der Genbank unter der Zugangsnummer KU321639 hinterlegt (Cunha et al., Genome Announcements, März/April 2016, Volume 4, Issue 2, S. 1-2).

In dem erfindungsgemäßen Verfahren werden also sowohl das non-structural protein 1, wie auch das envelope protein eines Zika-Virus eingesetzt.

In einer weiteren Ausführungsform wird auch als Antigen eingesetzt:

### f) NS1-Protein des Gelbfieber-Virus (YFV)

Das Genom des Gelbfieber-Virus kodiert für die drei strukturellen Proteine C, prM, E und in die sieben nicht-strukturellen Proteine NS1, NS2A, NS2B, NS3, NS4A, NS4B, NS5. Entsprechendes gilt auch für DENV und ZIKV. Erfindungsgemäß eingesetzt wird ein Peptid aus dem NS1-Protein oder ein Proteinfragment hiervon. Für Reisen in Gelbfieberendemie-Gebiete ist eine Impfung vorgeschrieben, die mit einem attenuierten Virusstamm durchgeführt wird. Es kann daher vorteilhaft sein, bei dem Verfahren auch das NS1-Protein des Gelbfiebervirus einzusetzen.

Erfindungsgemäß wurden einige bevorzugte Konstrukte von Fragmenten bzw. von immunologisch hochrelevanten Peptidfragmenten hergestellt, die bei dem Testverfahren und den Testkits besonders bevorzugt sind. Es handelt sich hierbei um folgende Konstrukte (Teilfragmente) des Envelope Proteins von jeweils Dengue- sowie Zika-Virus. Die getesteten Konstrukte weisen neben den Envelope Protein Sequenzen noch jeweils einen C-terminalen His-Tag und eine Protease-Schnittstelle auf (die Antigensequenzen von DENV und ZIKV sind unterstrichen):
- Domäne III des Envelope Proteins von Dengue-Virus Serotyp 2 (Stamm 16681) mit Protease-Schnittstelle und His-Tag:
- Domäne III des Envelope Proteins von Zika-Virus (Stamm SPH2015) mit Protease-Schnittstelle und His-Tag:

Diese Fragmente könnten in den Tests bevorzugt verwendet werden.

Bevorzugt eingesetzte Teilfragmente sind folgende NS1-Peptide, die zur Differenzierung eingesetzt werden können:
- Peptide des NS1 Proteins. Nachfolgend sind hier nur die Sequenzen für Dengue-Virus Serotyp 2 Stamm 16681; NS1 aufgeführt; entsprechende Sequenzen können ohne Schwierigkeiten in den Sequenzen von anderen Serotypen und Viren der Familie Flaviviridae aufgefunden werden:
   - **KRSLRPQPTELKYSWKTWGKAKMLSTESHNQT** (SEQ ID NO:3)
   - **PWHLGKLEMDFDFCD** (SEQ ID NO:4)
   - **DSGCVVSWKNKELKCGSGIFITDN** (SEQ ID NO:5).

Die oben wiedergegebenen bevorzugt verwendeten Fragmente mit den SEQ ID Nummern 3, 4 und 5 wurden deshalb ausgewählt, weil es sich hierbei um Fragmente der Antigene handelt, gegen die bevorzugt vom Immunsystem der infizierten Patienten Antikörper produziert werden. Diese Antikörper sind aber gut voneinander unterscheidbar, und zwar dahingehend, dass, wenn Antikörper gegen die angegebenen Fragmente des NS1-Proteins von Dengue-Virus vorhanden sind, diese nicht mit den homologen Regionen des NS1-Proteins reagieren, das vom Zika-Virus herstammt. Dies gilt auch umgekehrt. Hintergrund dieser Erkenntnis könnte sein, dass aufgrund der hohen Homologie zwischen Dengue- und Zika-Virus eine Differenzierung auf Grundlage von linearen Epitopen nicht oder nur schwer möglich ist. Da auch die dreidimensionale Struktur der beiden NS1-Antigene sehr ähnlich sein dürfte, wird erfindungsgemäß davon ausgegangen, dass der Hauptunterschied zwischen den ausgewählten Fragmenten des NS1 von Dengue-Virus und Zika-Virus in Ladungsunterschieden an der Oberfläche der beiden Proteine besteht.

Dieser Unterschied wird durch die oben aufgeführten bevorzugt verwendeten Fragmente zur Differenzierung ausgenutzt. Durch einen Vergleich der Sequenzen von Dengue- und Zika-NS1 und die Verwendung eines dreidimensionalen Modells können weitere derartige Sequenzen aufgefunden werden. Wenn Antigenfragmente eingesetzt werden, muss diesen Besonderheiten Rechnung getragen werden und es müssen solche Bereiche für die Peptide ausgewählt werden, die für unterschiedliche antigene Bereiche verantwortlich sind. Dies kann dazu führen, dass bevorzugt Volllängenproteine eingesetzt werden oder Proteine, die zu wenigstens 90 % den Volllängenproteinen entsprechen, es sei denn, immunologisch unterscheidbare Regionen können identifiziert werden.

In bevorzugter Ausführungsform werden Volllängenproteine eingesetzt, wobei die Differenzierung über den direkten Reaktivitätsvergleich von homologen Proteinen, also Dengue-NS1 gegenüber Zika-NS1, vorgenommen wird. Wenn beim E-Protein Fragmente eingesetzt werden, handelt es sich bevorzugt um die Domänen I bis III. Bei DENV-2 handelt es sich um die Aminosäuren 1-395 von insgesamt 495, bei ZIKV-E-Protein um die AS 1-404 von insgesamt 505 AS. Für die Objektivierung des Reaktivitätsvergleichs werden bevorzugt maschinelle Mittel, also Scans und eine geeignete Auswertungssoftware verwendet.

Die Antigene werden auf einer Festphase aufgebracht. Es gibt verschiedene Möglichkeiten, wie dies bewerkstelligt werden kann. In einer bevorzugten Ausführungsform werden die Antigene auf einem Trägerstreifen als Banden aufgebracht. Man spricht in diesem Fall von einem sogenannten Line-Assay. Alternativ hierzu können die Antigene, jeweils räumlich getrennt voneinander, auf ELISA-Testplatten aufgebracht werden, wobei sowohl ein Antigen in einem Reaktionsgefäß wie auch mehrere räumlich voneinander getrennt in einem Reaktionsgefäß eingebracht (Protein-Chip/array) und an die feste Phase gekoppelt werden können. Eine weitere Ausführungsform besteht darin, dass die Antigene an vorzugsweise magnetisierbare Kugeln gekoppelt werden. Dadurch können die Antigene bequem mit der Probe in Kontakt gebracht werden und wieder aus der Reaktionsmischung entfernt werden.

Nach der Reaktion der auf die feste Phase gekoppelten Antigene und der Probe bildet sich ein Immunkomplex aus dem Antigen und den spezifisch hieran bindenden Antikörpern. Um unspezifische Bindungen zu vermeiden, werden einerseits bei der Herstellung der Testkits all diejenigen Stellen abgesättigt, die eine unspezifische Bindung hervorrufen könnten. Es kann sich bei derartigen unspezifischen Bindungsstellen um Teile des Trägermaterials handeln. Nach der Reaktion müssen jedoch Antikörper, die nicht spezifisch gebunden wurden, abgetrennt werden. Dies erfolgt in bevorzugter Ausführungsform durch Waschen mit geeigneten Waschlösungen bzw. Puffern.

Auf der festen Phase hat sich durch die Reaktion des Antigens mit spezifischen, hiergegen gerichteten Antikörpern ein Immunkomplex gebildet, der in ein auswertbares Signal umgewandelt werden muss, sofern in der Probe spezifische Antikörper vorhanden sind. Für die Umwandlung des auf der Festphase gebildeten Immunkomplexes in ein auswertbares Signal gibt es verschiedene Möglichkeiten. Bevorzugt muss eine derartige Umwandlung in (semi) quantitativer Art und Weise erfolgen, weil es für die Auswertung des Testergebnisses erforderlich ist, dass man die relativen Stärken der Signale miteinander vergleichen kann.

In einer bevorzugten Ausführungsform wird der Immunkomplex auf der Festphase dadurch sichtbar gemacht, dass ein Anti-Antikörper zugegeben wird, der gekoppelt ist mit einem signalgebenden Mittel. Bei den Anti-Antikörpern handelt es sich üblicherweise um in Labortieren (beispielsweise Kaninchen, Ziege) erzeugte polyklonale Antikörper, die entweder gerichtet sind gegen humane IgG-Antikörper oder humane IgM-Antikörper. Die Anti-Antikörper sind üblicherweise gerichtet gegen den Fc-Teil der Antikörper aus der Patientenprobe, die an die jeweiligen Antigene gebunden haben.

Die Anti-Antikörper sind gekoppelt mit einem Signal erzeugenden Mittel. Hierbei kann es sich um ein Mittel handeln, das direkt ein Signal erzeugt, also beispielsweise einen Farbstoff oder einen Fluoreszenz-Farbstoff. Es kann sich aber auch um ein Mittel handeln, das das Signal verstärkt. In bevorzugter Ausführungsform handelt es sich hierbei um ein Enzym, wie beispielsweise Meerrettich-Peroxidase oder alkalische Phosphatase. Das Enzym wandelt ein Substrat in ein messbares Signal, beispielsweise ein gefärbtes Produkt, um. Je länger das Enzym auf das Substrat einwirken gelassen wird, desto stärker ist das gefärbte Signal.

Die Reaktion wird nach einer vorbestimmten Zeit dadurch beendet, dass beispielsweise gewaschen wird.

In dem erfindungsgemäßen Verfahren wird zur Abgrenzung des Rauschens, das immer bei immunologischen Tests auftreten kann, ein sogenannter "Cutoff' eingesetzt. Dieser Cutoff befindet sich beispielsweise auf dem Teststreifen und wird dadurch unter gleichen Bedingungen wie die zu überprüfenden Proben getestet. Der Cutoff des Tests bzw. die Intensität der Cutoff Bande wird so eingestellt, dass definierte negative Probenkollektive unterhalb des Cutoffs liegen und definierte positive Kollektive oberhalb des Cutoffs liegen.

Nach Durchführung des Tests und Umwandlung des Immunkomplexes in ein messbares Signal wird die Intensität des Signals bewertet. Hierbei wird die in Tabelle 1 wiedergegebene Beurteilung zugrundegelegt:

**Tabelle 1: Bewertungsschema**

| **Intensität des Signals** | **Bewertung** |
|---|---|
| keine Reaktion | - |
| Reaktivität unterhalb der Cutoff-Bande | +/- |
| Reaktivität gleich Cutoff-Bande | + |
| Reaktivität stärker als Cutoff-Bande | ++ |
| Reaktivität deutlich stärker als Cutoff-Bande | +++ |
| maximale Reaktivität | ++++ |

Die in Tabelle 1 wiedergegebene Bewertung stellt die visuelle Bewertung dar. Die Messung kann natürlich auch mit geeigneten Geräten, beispielsweise entsprechend eingestellten Photometern, durchgeführt werden. Dann kann die Intensität des Signals einem bestimmten, objektiv messbaren Wert zugeordnet werden.

In bevorzugter Ausführungsform wird das Testverfahren mit derselben Probe für den IgG- und IgM-Nachweis durchgeführt und es wird sowohl das Vorhandensein bzw. die Abwesenheit von IgM und IgG Antikörpern gegen die Antigene bestimmt. Ein hierfür geeigneter Testkit enthält alle hierfür benötigten Reagenzien.

Die Auswertung der Tests ist auch maschinell möglich, was zu objektiveren Ergebnissen führt. Dabei wird die Reaktionsintensität mit einer geeigneten Vorrichtung (Scanner etc.) gemessen und die erhaltenen Messwerte werden mit einem geeigneten Programm verrechnet.

In bevorzugter Ausführungsform werden bei dem erfindungsgemäßen Verfahren die Auswertungen der Teststreifen computergestützt mittels Teststreifenauswerte-Software durchgeführt. Hierbei werden die entwickelten Teststreifen per Scanverfahren eingelesen und die Signalstärken, gekennzeichnet durch die Farbintensität (Graustufen) der einzelnen Antigenbanden, gemessen. Dies ermöglicht einerseits den direkten Vergleich der Signalstärken zweier oder mehrerer Antigenbanden sowie andererseits den Vergleich der einzelnen Bandenintensitäten mit der Cutoff-Intensität. Im Gegensatz zur visuellen Auswertung kann das genaue Verhältnis von Bandenintensitäten zueinander oder zur Cutoff-Intensität (Cutoff-Index Wert) angegeben werden.

Das erfindungsgemäße Verfahren kann bevorzugt mit entsprechend angepassten Testkits durchgeführt werden. Diese Testkits beinhalten die oben aufgeführten Teststreifen mit den entsprechenden Antigenen, Kontrollbanden (u.a. Cutoff-Bande) und die Reagenzien zum Waschen der Teststreifen oder Test-Mikrotiterplatten. Außerdem enthalten die Testkits häufig Positiv- und Negativ-Kontrollseren, sowie Reaktionskontrollen.

Aufgrund der Schwierigkeiten, die der Differenzialdiagnose inhärent sind (hohe Ähnlichkeit der Antigene, Mehrfachinfektionen), sollten so viel wie mögliche Informationen zu der Auswertung herangezogen werden. Dazu gehört die Anamnese des Patienten, soweit verfügbar. Weiterhin kann es, je nach Ausgestaltung der spezifischen Situation, vorteilhaft sein, wenn sowohl die IgM-Antwort, wie auch die IgG-Antwort gegen dieselben Antigene durchgeführt werden. Dies kann beispielsweise einen Hinweis auf frische Infektionen oder länger zurückliegende Infektionen geben. Auch Mehrfachinfektionen können festgestellt werden.

In besonders bevorzugter Ausführungsform wird der Test als Line-Immuno-Assay durchgeführt. Bei diesem Testprinzip werden die Einzelantigene an speziellen Stellen separat auf den Teststreifen aufliniert, was die Identifizierung von spezifischen Antikörpern gegen die einzelnen Antigene von Dengue-, Chikungunya- und Zika-Viren ermöglicht. In einer besonders bevorzugten Ausführungsform enthält ein derartiger Line-Immuno-Assay die rekombinant hergestellten Volllängen-Antigene NS1 und E-Protein von Dengue-Virus, mindestens ein Chikungunya-Virus Antigen wie beispielsweise das E1-Protein und das NS1 und das E-Protein von Zika-Virus. Das E1-Protein von Chikungunya-Virus kann gegebenenfalls auch durch Virus-like Particles von Chikungunya-Virus ersetzt werden. Der erfindungsgemäße Test ergibt Reaktionen unterschiedlicher Stärke mit den bevorzugt eingesetzten Antigenen. Bei schwierigen Fällen kann die serologische Bewertung im Zusammenhang mit den Ergebnissen des IgG- und IgM-Tests erfolgen, wobei der Zeitpunkt der Infektion bzw. der Zeitpunkt der Probenentnahme nach Einsetzen klinischer Symptome und gegebenenfalls die Reisehistorie der zurückliegenden sechs Wochen berücksichtigt werden kann.

Bei primären Flavivirus-Infektionen werden normalerweise minimale Kreuzreaktivitäten zwischen den genetisch verwandten Flaviviren beobachtet.

Bei sekundären Flavivirus-Infektionen können hingegen vermehrt heterologe, d.h. mit anderen Flaviviren kreuzreagierende Antikörper gebildet werden. Kreuzreagierende Antiköper treten verstärkt zwischen Dengue- und Zika-Virus auf. Aber auch Seren von Patienten mit einer vorausgegangenen Infektion oder mit Impfung gegen andere Flaviviren (wie Gelbfiebervirus, West-Nil-Virus, Frühsommermeningitis-Virus, St. Louis Enzephalitis-Virus oder japanischer Enzephalitis-Virus) können in serologischen Tests Kreuzreaktionen hervorrufen.

In der Regel ist die IgM-Immunantwort gegen Flaviviren spezifischer im Vergleich zur IgG-Immunantwort. Dies gilt insbesondere für eine Flaviviren-Sekundärinfektion, d.h. wenn der Patient bereits eine oder mehrere Flavivirus-Infektion(en) hatte. Da die IgM-Antwort bei einer Sekundärinfektion jedoch i.d.R. deutlich niedriger ausfällt oder gar nicht nachweisbar ist, ist es von Vorteil, wenn mittels IgG-Immunantwort ein Hinweis auf den Krankheitserreger erhalten werden kann. Über den direkten Vergleich der Antikörperantworten gegen z.B. NS1 im IgG-Test ergeben sich oftmals Hinweise auf den Infektionserreger. Hierbei ist aber zu beachten, dass bei -akuten Infektionen die IgG-Antwort gegen den aktuellen Erreger i.d.R. noch nicht erfolgt ist. Das heißt, bei Proben aus der Akutphase ist hier eine Folgeprobe im Abstand von bevorzugt 1-2 Wochen notwendig. Dies wird in Beispiel 1, Patient 4 gezeigt.

Eine Differenzierung zwischen Dengue- und Zika-Virus ist bevorzugt anhand der spezifischen NS1-Antigenreaktivitäten möglich. Eine Mehrfachinfektion mit Dengue-Virus sollte nicht zu einer gleich starken Reaktivität von Dengue- und Zika-NS1-Antigen führen.

Wenn gleich stark reaktive Banden von Dengue- und Zika-Virus-E-Protein bei gleichzeitig fehlender Reaktivität mit NS1 von Dengue- und Zika-Virus vorliegen, kann keine sichere Flavivirus-Differenzierung erfolgen. Wurde die Probe nur wenige Tage nach Auftreten der Symptome abgenommen, kann es sinnvoll sein, eine Folgeprobe zu testen. Insbesondere eine zu diesem frühen Zeitpunkt vorhandene schwache IgG-Reaktivität mit beiden (DENV und ZIKV) E-Proteinen kann auf eine vorangegangene Infektion mit einem anderen Flavivirus oder eine Flavivirus-Impfung zurückzuführen sein. Die Ausbildung von spezifischen Anti-Dengue- bzw. Anti-Zika-Virus-Antikörpern, insbesondere gegen das NS1-Antigen, kann noch erfolgen.

Wenn wenigstens eine antigene Bande ausgewählt aus Dengue-Virus-NS1, Dengue-Virus-E-Protein, Zika-Virus-NS1 sowie Zika-Virus-E-Protein deutlich positiv reagiert, liegt eine Immunantwort gegen Flavivirus-Infektion vor. Wenn keine Bande reagiert oder nur Banden mit sehr schwacher Intensität unterhalb des Cutoffs aufgefunden werden, ist das Ergebnis für Dengue- und Zika-Virus negativ.

Für die Differenzierung zwischen Dengue-Virus und Zika-Virus mittels NS1 gilt Folgendes:

| **Probenahme nach Einsetzen klinischer Symptome** | **IgG und/oder IgM NS-1 Antigenmuster** | **Interpretation** |
|---|---|---|
| ≥ größer/gleich 4-6 Tage | DENV NS-1 Bande reagiert mit derselben (+) oder stärkeren Intensität wie die Cutoff-Bande und ist deutlich sichtbar stärker gefärbt als die ZIKV NS-1 Bande | Flavivirus positiv, Verdacht auf Dengue |
| ≥ größer/gleich 4-6 Tage | DENV NS-1 UND ZIKV NS-1 Banden reagieren mit derselben (+) oder stärkeren Intensität wie die Cutoff-Bande und sind, ohne deutlich sichtbaren Unterschied, gleich stark gefärbt. | Flavivirus positiv, keine Differenzierung, positives NS-1 ist spezifisch für DENV UND ZIKV |
| ≥ größer/gleich 4-6 Tage | ZIKV NS-1 Bande reagiert mit derselben (+) oder stärkeren Intensität wie die Cutoff-Bande und ist deutlich sichtbar stärker gefärbt als die DENV NS-1 Bande. | Flavivirus positiv, Verdacht auf Zika |

Für den Nachweis des Chikungunya-Virus gilt Folgendes:

| | |
|---|---|
| Die CHIKV E1-Antigenbande reagiert mit derselben (+) oder stärkeren Intensität wie die Cutoff-Bande | Chikungunya positiv |
| Keine CHIKV E1-Antigenbande (-) oder CHKV E1-Antigenbande mit schwächerer (+/-) Intensität als die Cutoff-Bande | Chikungunya negativ |

Betont werden sollte, dass das erfindungsgemäße Testverfahren eindeutige, messbare Reaktionsunterschiede ergibt, die aufgrund der Komplexität der Fragestellung im Gesamtzusammenhang interpretiert werden sollten.

Die Erfindung wird in den Beispielen weiter erläutert:

### Beispiel 1

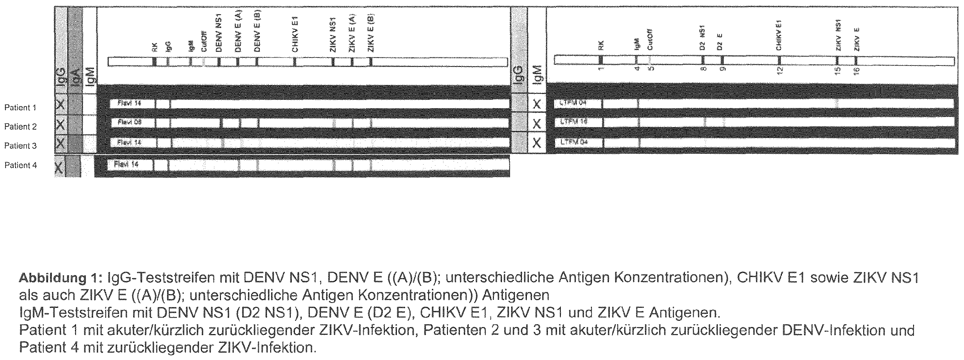

Es wurde ein sogenannter "Line-Assay"-Teststreifen mit den erfindungsgemäß eingesetzten Antigenen hergestellt, wobei die einzelnen Antigene an unterschiedlichen Stellen des Teststreifens aufgebracht wurden. Diese Teststreifen wurden mit Seren von vier verschiedenen Patienten umgesetzt. Die Ergebnisse sind in Abbildung 1 dargestellt. Hierbei handelte es sich um:
Patient 1: Der Patient zeigte keinerlei IgG Antikörperreaktionen gegen Dengue-, Chikungunya- und Zika-Virus, aber IgM Antikörper gegen das ZIKV NS1 Protein. Damit hat der Patient eine frische oder kürzlich zurückliegende Zika-Virus-Infektion. Die Infektion ist relativ einfach nachzuweisen, da es sich um eine singuläre, Flavivirus-Erstinfektion handelt.
Patient 2: Der Patient hat sowohl IgG Antikörper gegen Dengue- als auch gegen Zika-Virus. Im direkten Vergleich der korrespondierenden Antikörper gegen DENV/ZIKV NS1 stellt sich dar, dass der Antikörpertiter von Dengue-Virus NS1 deutlich stärker ist (++++) als der korrespondierende von Zika-Virus NS1 (++). Das gleiche gilt für die korrespondierenden E-Proteine von Dengue- (+++) und Zika-Virus (++). Im direkten Vergleich der IgG Antikörpertiter ist es daher möglich, eine Unterscheidung zwischen anti-Dengue- und anti-Zika Virus IgG-Antikörpern vorzunehmen. Des Weiteren weist der Patient IgM-Antikörper, die ausschließlich gegen die Dengue-Virus Antigene gerichtet sind, auf. Anhand der IgM-Antwort (Dengue-Virus NS1 (++), Dengue-Virus E (++)) lässt sich die Aussage treffen, dass der Patient eine akute bzw. kürzlich zurück liegende DENV-Infektion hatte.
Patient 3: Der Patient hat sowohl IgG Antikörper gegen Dengue- als auch Antikörper gegen Zika-Virus. Im direkten Vergleich der korrespondierenden Antikörper gegen DENV/ZIKV NS1 stellt sich dar, dass der Antikörpertiter von Dengue-Virus NS1 deutlich höher (stärker) ist (++) als der korrespondierende von Zika-Virus NS1 (+/-). Anhand der korrespondierenden E Proteine von Dengue-Virus (+) und Zika-Virus (+) kann nicht differenziert werden. Auch hier ist es möglich, im Vergleich der anti-NS1 IgG Antikörpertiter eine Aussage zu treffen. Weiterhin weist der Patient IgM Antikörper gegen das NS1 Protein von Dengue-Virus (++) auf. Der Patient hat eine akute bzw. kürzlich zurück liegende DENV-Infektion.
Patient 4: Der Patient hat keine IgG Antikörper gegen Dengue-Virus NS1 (-), aber Antikörper gegen Zika-Virus NS1 (++). Die Antikörper gegen DENV E (++) als auch ZIKV E lassen keine Differenzierung zu. Durch die gegen NS1 gerichteten Antikörper ist es hier möglich, auf eine Zika-Virus Infektion zu schließen. Der IgM-Teststreifen, hier nicht dargestellt, ist allerdings negativ. Dies lässt vermuten, dass es sich um eine zurückliegende Infektion handelt.

Zusammenfassend wurde die Auswertung des Tests in der nachfolgenden Tabelle 2 dargestellt:
Auswertung: die verschiedenen Intensitäten der Banden sind mit -, +/-, +, ++, +++, ++++ dargestellt. RK ist eine Reaktionskontrolle, die erscheinen muss. IgG oder IgM gibt die jeweilige Immunglobulin Klasse an. Die Banden werden immer im Vergleich zur Cutoff Bande ausgewertet. DENV NS1, DENV E (A), DENV E (B), CHIKV E1, ZIKV NS1, ZIKV E (A) und ZIKV E (B). Variante (A) und (B) entsprechen jeweils zwei unterschiedlichen Antigenkonzentrationen.

**Tabelle 2: Zusammenfassung Auswertung Patienten 1-4. n.a. ist die Abkürzung für "not available" und weist darauf hin, dass der IgM-Teststreifen die entsprechende Antigenbande nicht enthält.**

| Patient | IgG/M | DENV NS1 / D2 NS1 | DENV E (A) / D2 E | DENV E (B) / n.a. | CHIKV E1 / CHIKV E1 | ZIKV NS1 / ZIKV NS1 | ZIKV E (A) / ZIKV E | ZIKV E (B) / n.a. |
|---|---|---|---|---|---|---|---|---|
| 1 | IgG | - | - | - | - | - | - | - |
| 1 | IgM | - | - | n.a. | - | ++ | - | n.a. |
| 2 | IgG | ++++ | +++ | +++ | - | ++ | ++ | ++ |
| 2 | IgM | ++ | ++ | n.a. | - | - | - | n.a. |
| 3 | IgG | ++ | + | + | - | +/- | + | + |
| 3 | IgM | ++ | - | n.a. | - | - | - | n.a. |
| 4 | IgG | - | ++ | ++ | - | ++ | ++ | ++ |
| 4 | IgM | n.a. | | | | | | |

### Beispiel 2

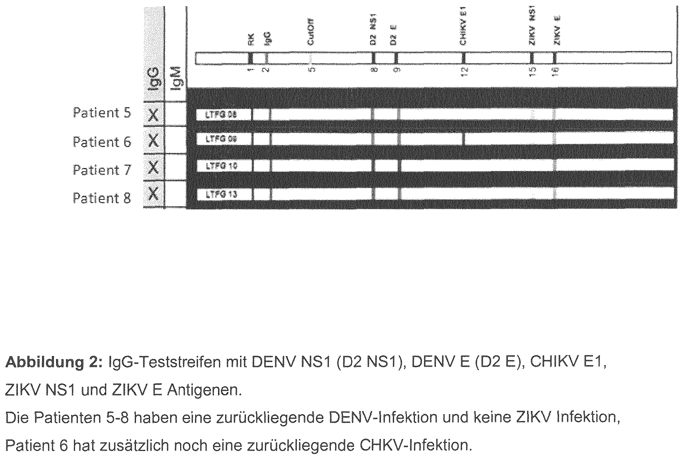

Es wurde weiterhin ein Line-Assay mit Seren von Patienten durchgeführt, bei denen eine Differenzierung zwischen Dengue-Virus und Zika-Virus durch einen Vergleich der Intensität der Banden möglich ist. Die Ergebnisse sind in Abbildung 2 dargestellt.

Die Abbildung 2 enthält Beispielstreifen mit Proben von Patienten 5-8, bei denen eine Differenzierung zwischen DENV und ZIKV im IgG-Test durch direkten Vergleich der DENV und ZIKV NS1-Bandenintensität möglich ist. Die IgM-Testergebnisse sind negativ, was einen Hinweis auf zurückliegende Infektionen gibt.

Sowohl DENV als auch ZIKV NS1 sind positiv (reagieren stärker im Vergleich zur Cutoff-Bande). Aber anhand des direkten Intensitätsvergleichs beider Antigene kann man erkennen, dass auf den Teststreifen die jeweilige DENV NS1-Bande deutlich stärker gefärbt ist im Vergleich zur ZIKV NS1-Bande.

Die Patienten 5-8 haben eine zurückliegende DENV-Infektion und keine ZIKV-Infektion, Patient 6 hat zusätzlich noch eine zurückliegende CHIKV-Infektion.

### Beispiel 3

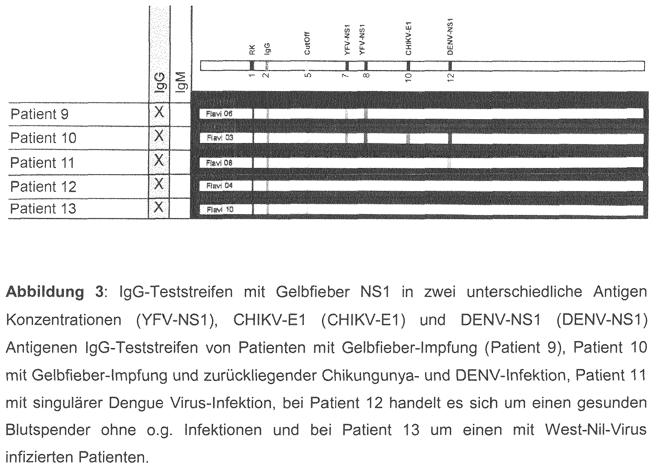

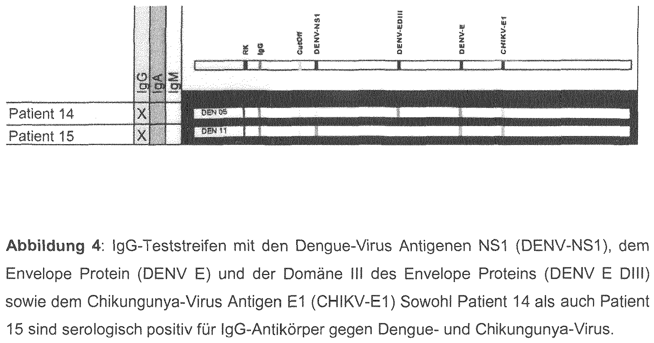

Die Abbildung 3 enthält Beispielstreifen mit Proben von Patienten 9-13, bei denen eine Differenzierung zwischen Gelbfieber-, Chikungunya-, Dengue- und West-Nil-Virus möglich ist.

Patient 9 wurde innerhalb der letzten zwei Jahre gegen Gelbfieber geimpft und zeigt entsprechend im IgG-Nachweis eine Reaktion mit dem Gelbfieber-Virus NS1 Antigen, es liegt keinerlei Kreuzreaktivität zu anderen Erregern vor. Patient 10 ist gegen Gelbfieber geimpft und weist des Weiteren IgG-Antikörper gegen Chikungunya- und Dengue-Virus auf. Es reagieren die NS1 Antigene des Gelbfieber- und des Dengue-Virus sowie das E1 Antigen des Chikungunya-Virus. Patient 11 ist ein Beispiel für eine zurückliegende singuläre Dengue-Virus Infektion (Patient mit Dengue-Fieber vor 7 Jahren) und man sieht, dass keinerlei Kreuzreaktivität zu Gelbfieber- (Flavivirus) und Chinkungunya-Virus vorliegt. Bei Patient 12 handelt es sich um einen gesunden Blutspender aus Deutschland ohne o.g. Infektionen, welcher als Negativkontrolle fungiert.
Bei Patient 13 handelt es sich um einen mit West-Nil-Virus infizierten Patienten, er zeigt keinerlei Kreuzreaktivität zu den anderen Erregern.

### Beispiel 4

Es wurden zwei Teststreifen hergestellt, die einerseits das Dengue-Virus (Serotyp 2) Envelope Protein (DENV E) sowie ein Teilfragment des Envelope Proteins bestehend aus der Domäne III (DENV EDIII) enthielten. Die Teststreifen wurden umgesetzt mit Serum der Patienten 14 und 15, wobei beide Patienten serologisch positiv sind für IgG-Antikörper gegen Dengue- und Chikungunya-Virus. Das Beispiel belegt, dass der Test nicht nur mit den Volllängen-Antigenen, sondern auch mit geeigneten Fragmenten durchgeführt werden kann.

### Beispiel 5

Entsprechend Beispiel 2 wurden Line-Assay Teststreifen zum Nachweis von Antikörpern gegen Dengue-Virus NS1 und E-Protein, Chikungunya-Virus E1-Protein sowie Zika-Virus NS1 und E-Protein hergestellt und mit Patientenproben getestet.

Das erste Patientenkollektiv (a) umfasst Patienten, die nach Aufenthalt in einem Endemiegebiet für einen oder mehrere der drei genannten Erreger nach Deutschland zurückgekehrt sind. Das zweite Patientenkollektiv (b) umfasst Patienten aus einem Dengue-Virus Endemiegebiet in Lateinamerika, die IgG-Antikörper gegen Dengue-Virus aufweisen.

Die Auswertung der Ergebnisse wurde mit der automatischen Teststreifen-Auswertesoftware recomScan durchgeführt. Analog zu Beispiel 2 konnte die serologische Differenzierung zwischen einer Dengue- und einer Zika-Virus-Infektion anhand des direkten Vergleichs der DNEV-NS1- und ZIKV NS1-Bandenintensitäten durchgeführt werden.

### Tabelle 3

Die Tabellen 3a und 3b enthalten zum einen die Graustufenwerte der DENV-NS1 sowie ZIKV-NS1 Banden sowie die Cutoff-Index-Werte (COI; Wert des Signals in Relation zum Cutoff gesetzt) der einzelnen Antigenbanden. Entsprechend der visuellen Auswertung wird ein COI-Wert ≥ 1 als positives Signal gewertet. Die Differenzierung zwischen DENV und ZIKV erfolgt nur dann, wenn DENV NS1 und/oder ZIKV NS1 einen COI-Wert ≥ 1 besitzen und eines der beiden NS1 Banden mindestens die zweifache Intensität (zweifache Graustufenwerte) der anderen NS1 Bande aufweist.
(a) Patientenkollektiv Reiserückkehrer (kürzlich zurückliegende Infektionen):
   Patient 16 und 19: Dengue-Virus positiver Vorbefund (Nukleinsäurenachweis) Patient 17 und 18: Zika-Virus positiver Vorbefund (Nukleinsäurenachweis) Von Patient 18 und 19 wurden jeweils eine Probe im Anschluss an die Konvaleszenz-Phase nach kürzlich zurückliegender Infektion und eine Folgeprobe nach mehreren Monaten getestet. Die Ergebnisse der jeweils ersten Abnahme sind in Tabelle 3a jeweils in der oberen Zeile angegeben, die der Folgeprobe jeweils in der darunterliegenden Zeile.
   Patient 16 hatte bereits in der Vergangenheit eine Dengue-Virus-Infektion. Typischerweise ist der IgM-Titer in Folge einer Sekundärinfektion deutlich niedriger im Vergleich zur Primärinfektion und die IgG-Antwort weist eine höhere Flavivirus-Kreuzreaktivität auf. Trotzdem kann serologisch im IgG-Test zwischen einer Antikörperantwort gegen Dengue- und Zika-Virus anhand des direkten NS1-Vergleichs beider Erreger unterschieden werden. In dem genannten Beispiel wird auf dieser Grundlage eine IgG-Immunantwort gegen Dengue-, nicht aber gegen Zika-Virus nachgewiesen. Somit kann aufgrund des IgG-Testergebnisses eine kürzlich zurückliegende Zika-Virus-Infektion ausgeschlossen werden. Patienten 17 und 18 (erste Abnahme) sind IgG- und IgM-seropositiv für ZIKV. Mehrere Monate nach der Infektion weist die Folgeprobe von Patient 18 wie erwartet nur noch Anti-ZIKV IgG-Antikörper auf. Die erste Abnahme von Patient 18 ist IgG- und IgM-, die zweite nur IgG-positiv für ZIKV. Patienten 19 (erste Abnahme) ist IgG- und IgM-seropositiv für DENV. Mehrere Monate nach der Infektion weist die Folgeprobe von Patient 19 wie erwartet nur noch Anti-DENV IgG-Antikörper auf.
   (a) 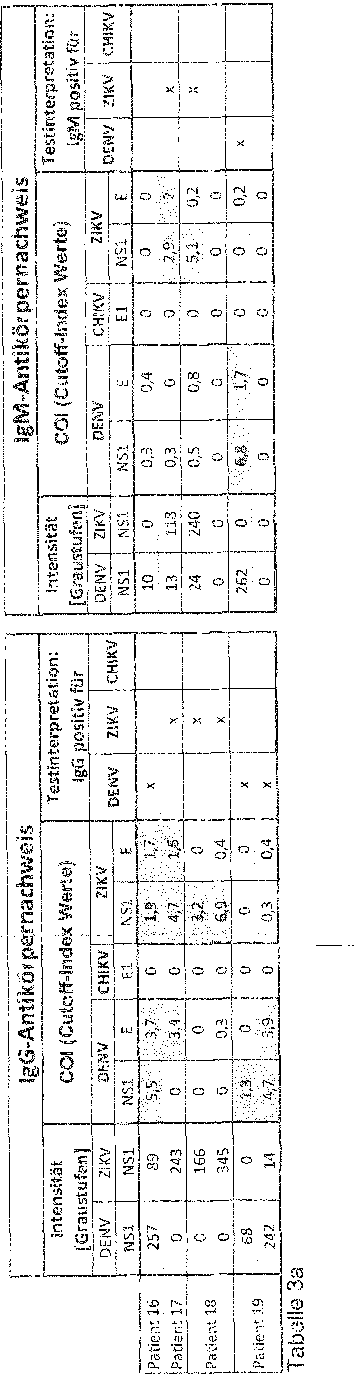
(b) Beispiele für Proben aus Dengue-Virus Endemiegebieten. Es wurden Proben ausgewählt, die einen DENV IgG-Titer aufweisen. 13/23 Proben zeigen sowohl ein DENV NS1- als auch ein ZIKV-NS1-Signal über dem Cut-off. Anhand des direkten NS1 Vergleichs ist jedoch eine Differenzierung möglich.
   (b)

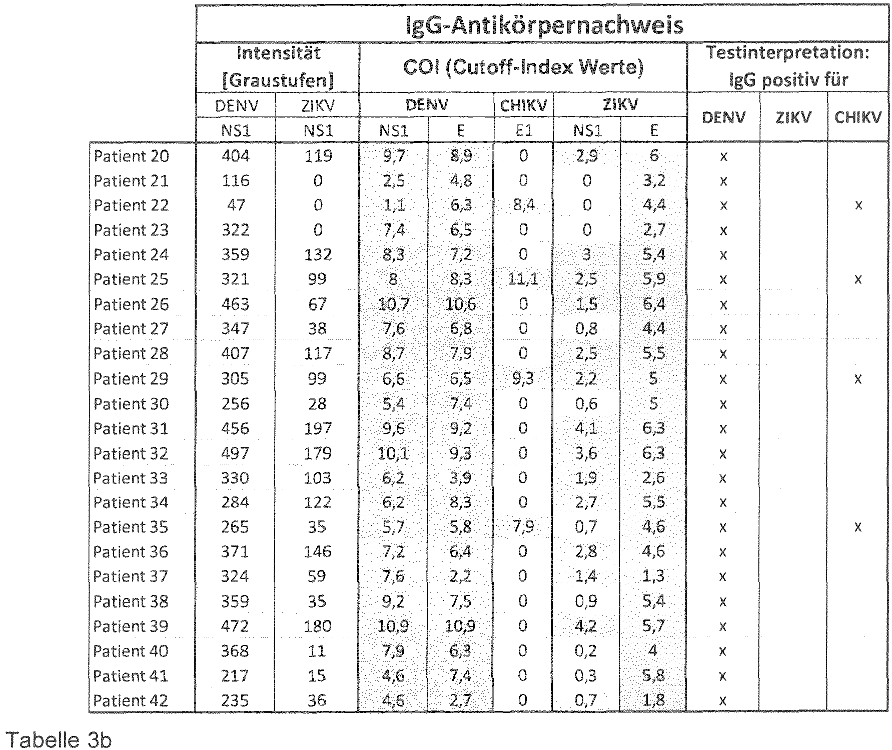

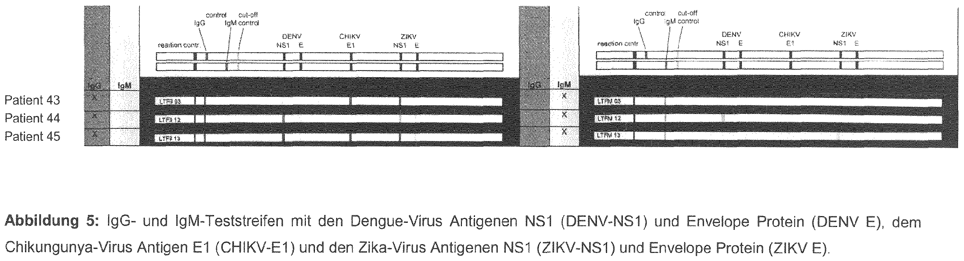

Die Ergebnisse eines Tests zeigt Abbildung 5: IgG- und IgM-Teststreifen mit DENV NS1, DENV E, CHIKV E1, ZIKV NS1 und ZIKV E Antigenen wurden eingesetzt. Der Patient 43 hat eine zurückliegende ZIKV und CHIKV Infektion. Patient 44 hat eine akute/kürzlich zurückliegende Dengue-Virus-Infektion (IgG und IgM positiv für DENV). Patient 45 ist IgG positiv für DENV, ZIKV und CHIKV. Die akute/kürzlich zurückliegende Infektion ist jedoch eine Infektion mit Zika-Virus, da für diesen Erreger noch Anti-ZIKV NS1 IgM-Antikörper nachweisbar sind.

## Patentansprüche

1. Verfahren zur immunologischen Differentialdiagnose einer Probe eines Patienten mit einer potentiellen Infektion eines Arbovirus, worin
a) eine Probe mit mehreren voneinander getrennt auf einer Festphase aufgebrachten Antigenen in Kontakt gebracht wird, wobei wenigstens folgende Antigene eingesetzt werden:
aa) das "non-structural protein 1" eines ersten Arbovirus oder ein immunologisch reaktives Teil hiervon mit wenigstens 8 Aminosäuren,
bb) ein "envelope protein" eines ersten Arborvirus oder ein immunologisch reaktives Teil hiervon mit wenigstens 8 Aminosäuren,
cc) ein "envelope protein " eines zweiten Arbovirus oder ein immunologisch reaktives Teil hiervon mit wenigstens 8 Aminosäuren,
dd) das "non-structural protein 1" eines dritten Arbovirus oder ein immunologisch reaktives Teil hiervon mit wenigstens 8 Aminosäuren und
ee) ein "envelope protein" eines dritten Arbovirus oder ein immunologisch reaktives Teil hiervon mit wenigstens 8 Aminosäuren;
b) Waschen der Festphase, um unspezifische Bindungen abzutrennen,
c) Umwandeln des auf der Festphase gebildeten Immunkomplexes in ein Signal und
d) Auswerten des Testverfahrens durch Vergleich der relativen Signalstärken,
**dadurch gekennzeichnet,dass** das erste Arbovirus ein Dengue-Virus Serotyp 2, das zweite Arbovirus ein Chikungunya-Virus, das dritte Arbovirus ein Zika-Virus ist

2. Verfahren nach Anspruch 1, **dadurch gekennzeichnet, dass** in dem Diagnoseverfahren weiterhin das folgende Antigen eingesetzt wird:
ff) ein Peptid mit wenigstens 8 Aminosäuren aus der NS1-Region eines vierten Arbovirus, wobei das vierte Arbovirus ein Gelbfieber-Virus ist.

3. Verfahren nach einem der Ansprüche 1 bis 2, **dadurch gekennzeichnet, dass** die Umwandlung des auf der Festphase gebildeten Immunkomplexes in ein Signal durch Anti-Antikörper erfolgt, die gekoppelt sind mit einem Signal-erzeugenden Mittel.

4. Verfahren nach Anspruch 3, **dadurch gekennzeichnet, dass** die Anti-Antikörper humane Anti-IgG-Antikörpar und/oder humane Anti-IgM-Antikörper sind.

5. Verfahren nach einem der Ansprüche 3 oder 4, **dadurch gekennzeichnet, dass** das farbgebende Mittel ein Enzym ist, das ein Substrat in ein gefärbtes Produkt umwandelt, das visuell sichtbar ist und/oder mit einer geeigneten Vorrichtung quantitativ messbar ist.

6. Verfahren nach einem der Ansprüche 1 bis 5, **dadurch gekennzeichnet, dass** für jede Probe bestimmt wird, ob die Antikörper zu der Klasse der IgM, der IgG oder zu beiden gehören.

7. Verfahren nach einem der vorhergehenden Ansprüche, **dadurch gekennzeichnet, dass** die Auswertung der Teststreifen durch einen Vergleich der Intensitäten der Signale der einzelnen Antigene erfolgt, wobei die einzelnen Antigene auf einen "Cutoff" referenziert werden und wobei für jedes Antigen die Intensität des Signals bestimmt wird, wobei folgende Beurteilung zugrundegelegt wird:
| **Intensität des Signals** | **Bewertung** |
|---|---|
| keine Reaktion | - |
| Reaktivität unterhalb der Cutoff-Bande | +/- |
| Reaktivität gleich Cutoff-Bande | + |
| Reaktivität stärker als Cutoff-Bande | ++ |
| Reaktivität deutlich stärker als Cutoff-Bande | +++ |
| maximale Reaktivität | ++++ |

8. Verfahren nach einem der vorhergehenden Ansprüche, **dadurch gekennzeichnet, dass** es sich um einen ELISA-Test handelt, wobei in jedes Reaktionsgefäß ein Antigen eingebracht wird, oder dass es sich um einen Line-Assay handelt und dass die Farbintensität mit einer Messvorrichtung quantitativ bestimmt und mit Hilfe einer Auswertvorrichtung das Verhältnis der Intensität der einzelnen Reaktionen In Bezug auf den Cut-Off-Wert bestimmt wird.

9. Testkit zur Durchführung eines Verfahrens nach einem der Ansprüche 1 bis 8, **dadurch gekennzeichnet, dass** das Testkit mehrere voneinander räumlich getrennte Festphasen beinhaltet, wobei das Testkit wenigstens folgende Antigene aufweist:
aa) das "non-structural protein 1" eines ersten Arbovirus oder ein Immunologisch reaktives Teil hiervon mit wenigstens 8 Aminosäuren,
bb) ein "envelope protein" eines ersten Arbovirus oder ein immunologisch reaktives Teil hiervon mit wenigstens 8 Aminosäuren,
cc) ein "envelope protein" eines zweiten Arborvirus oder ein immunologisch reaktives Teil hiervon mit wenigstens 8 Aminosäuren,
dd) das "non-structural protein 1" eines dritten Arbovirus oder ein immunologisch reaktives Teil hiervon mit wenigstens 8 Aminosäuren und
ee) ein "envelope protein" eines dritten Arbovirus oder ein immunologisch reaktives Teil hiervon mit wenigstens 8 Aminosäuren
sowie Reagenzien zum Waschen des Immunkomplexes und Reagenzien zur Umwandlung des Immunkomplexes in ein optisch sichtbares Signal und ein voreingestelltes Serum, das als Cutoff-Probe eingesetzt wird, **dadurch gekennzeichnet, dass** das erste Arbovirus ein Dengue-Virus Serotyp 2, das zweite Arbovirus ein Chikungunya-Virus, das dritte Arbovirus ein Zika-Virus ist.

10. Testkit nach Anspruch 9, **dadurch gekennzeichnet, dass** das Testkit weiterhin das folgende Antigen aufweist:
ff) ein Peptid mit wenigstens 8 Aminosäuren aus der NS1-Region eines vierten Arbovirus, wobei das vierte Arbovirus ein Gelbfieber-Virus ist.

11. Verfahren nach einem der Ansprüche 1 bis 8sowie Testkit nach Anspruch 9 oder 10, **dadurch gekennzeichnet, dass** der immunologisch reaktive Teil der Antigene wenigstens 40 Aminosäuren aufweist.

12. Verfahren nach einem der Ansprüche 1 bis 8 sowie Testkit nach einem der Ansprüche 9-10, **dadurch gekennzeichnet, dass** der immunologisch reaktive Teil der eingesetzten Antigene wenigstens 80 % der vollen Länge des natürlich auftretenden Antigens umfasst.

13. Verfahren nach einem der Ansprüche 1 bis 8 sowie Testkit nach einem der Ansprüche 9-10, **dadurch gekennzeichnet, dass** der reaktive Teil der eingesetzten Antigene wenigstens 95 % der vollen Länge des natürlich auftretenden Antigens umfasst.

## Claims

1. A method for the immunological differential diagnosis of a sample from a patient with a potential infection with an arbovirus, wherein
a) a sample is brought into contact with a plurality of antigens that, separated from each other, are applied to a solid phase, wherein at least the following antigens are used:
aa) the "non-structural protein 1" of a first arbovirus or an immunologically reactive part thereof having at least 8 amino acids,
bb) an "envelope protein" of a first arbovirus or an immunologically reactive part thereof having at least 8 amino acids,
cc) an "envelope protein" of a second arbovirus or an immunologically reactive part thereof having at least 8 amino acids,
dd) the "non-structural protein 1" of a third arbovirus or an immunologically reactive part thereof having at least 8 amino acids and
ee) an "envelope protein" of a third arbovirus or an immunologically reactive part thereof having at least 8 amino acids,
b) the solid phase is washed to separate non-specific bindings,
c) the immune complex formed on the solid phase is converted into a signal and
d) the test method is evaluated by comparing the relative signal strengths,
**characterized in that** the first arbovirus is a dengue virus serotype 2, the second arbovirus is a Chikungunya virus, the third arbovirus is a Zika virus.

2. The method according to claim 1, **characterized in that** in the diagnosis method further the following antigen is used:
ff) a peptide having at least 8 amino acids from the NS1 region of a fourth arbovirus, wherein the fourth arbovirus is a yellow fever virus.

3. The method according to one of claims 1 to 2, **characterized in that** the conversion of the immune complex formed on the solid phase to a signal is by anti-antibodies that are coupled to a signal-generating agent.

4. The method according to claim 3, **characterized in that** the anti-antibodies are human anti-IgG antibodies and/or human anti-IgM antibodies.

5. The method according to one of claims 3 or 4, **characterized in that** the coloring agent is an enzyme that converts a substrate into a colored product that is visually visible and/or can be quantitatively measured with a suitable device.

6. The method according to one of claims 1 to 5, **characterized in that** it is determined for each sample whether the antibodies belong to the class of IgM, IgG or both.

7. The method according to any of the preceding claims, **characterized in that** the evaluation of the test strips is made by comparing the intensities of the signals of the individual antigens, wherein the individual antigens are related to a "cut-off" and wherein for each antigen the intensity of the signal is determined, wherein the following assessment is taken as a basis:
| **Intensity of the Signal** | **Evaluation** |
|---|---|
| no reaction | - |
| reactivity below the cut-off band | +/- |
| reactivity equal to the cut-off band | + |
| reactivity stronger than the cut-off band | ++ |
| reactivity significantly stronger than the cut-off band | +++ |
| maximum reactivity | ++++ |

8. The method according to any of the preceding claims, **characterized in that** it is an ELISA test, wherein an antigen is placed in each reaction vessel, or it is a line assay and that the color intensity is quantitatively determined with a measuring device and the ratio of the intensity of the individual reactions with respect to the cut-off value is determined by means of an evaluation device.

9. A test kit for carrying out a method according to one of claims 1 to 8, **characterized in that** the test kit contains a plurality of spatially separated solid phases, wherein the test kit has at least the following antigens:
aa) the "non-structural protein 1" of a first arbovirus or an immunologically reactive part thereof having at least 8 amino acids,
bb) an protein" of a first arbovirus or an immunologically reactive part thereof having at least 8 amino acids,
cc) an protein" of a second arbovirus or an immunologically reactive part thereof having at least 8 amino acids,
dd) the "non-structural protein 1" of a third arbovirus or an immunologically reactive part thereof having at least 8 amino acids and
ee) an protein" of a third arbovirus or an immunologically reactive part thereof having at least 8 amino acids,
as well as reagents for washing the immune complex and reagents for converting the immune complex into an optically visible signal and a pre-adjusted serum that is used as the cut-off sample, **characterized in that** the first arbovirus is a dengue virus serotype 2, the second arbovirus is a Chikungunya virus, the third arbovirus is a Zika virus.

10. The test kit according to claim 9, **characterized in that** the test kit further has the following antigen:
ff) a peptide having at least 8 amino acids from the NS1 region of a fourth arbovirus, wherein the fourth arbovirus is a yellow fever virus.

11. The method according to one of claims 1 to 8 and the test kit according to claim 9 or 10, **characterized in that** the immunologically reactive part of the antigens has at least 40 amino acids.

12. The method according to one of claims 1 to 8 and the test kit according to one of claims 9-10, **characterized in that** the immunologically reactive part of the employed antigens comprises at least 80% of the full length of the naturally occurring antigen.

13. The method according to one of claims 1 to 8 and the test kit according to one of claims 9-10, **characterized in that** the reactive part of the employed antigens comprises at least 95% of the full length of the naturally occurring antigen.

## Revendications

1. Procédé de diagnostic immunologique différentiel d'un échantillon d'un patient présentant une infection à arbovirus potentielle, dans lequel
a) un échantillon est mis en contact avec plusieurs antigènes appliqués séparément les uns des autres sur une phase solide, dans lequel au moins les antigènes suivants sont utilisés :
aa) la « protéine non structurale 1 » d'un premier arbovirus ou une partie immunologiquement réactive de celle-ci présentant au moins 8 acides aminés,
bb) une « protéine d'enveloppe » d'un premier arbovirus ou une partie immunologiquement réactive de celle-ci présentant au moins 8 acides aminés,
cc) une « protéine d'enveloppe » d'un deuxième arbovirus ou une partie immunologiquement réactive de celle-ci présentant au moins 8 acides aminés,
dd) la « protéine non structurale 1 » d'un troisième arbovirus ou une partie immunologiquement réactive de celle-ci présentant au moins 8 acides aminés et
ee) une « protéine d'enveloppe » d'un troisième arbovirus ou une partie immunologiquement réactive de celle-ci présentant au moins 8 acides aminés ;
b) lavage de la phase solide pour séparer des liaisons non spécifiques,
c) conversion du complexe immun formé sur la phase solide en un signal et
d) interprétation du procédé de test par comparaison des intensités de signal relatives,
**caractérisé en ce que** le premier arbovirus est un virus de la dengue de sérotype 2, le deuxième arbovirus est un virus du chikungunya, le troisième arbovirus est un virus du zika.

2. Procédé selon la revendication 1, **caractérisé en ce que** dans le procédé de diagnostic, l'antigène suivant est en outre utilisé :
ff) un peptide présentant au moins 8 acides aminés de la région NS1 d'un quatrième arbovirus, dans lequel le quatrième arbovirus est un virus de la fièvre jaune.

3. Procédé selon l'une quelconque des revendications 1 à 2, **caractérisé en ce que** la conversion du complexe immun formé sur la phase solide en un signal s'effectue par des anti-anticorps qui sont couplés à un milieu générant un signal.

4. Procédé selon la revendication 3, **caractérisé en ce que** les anti-anticorps sont des anticorps anti-IgG humains et/ou des anticorps anti-IgM humains.

5. Procédé selon l'une quelconque des revendications 3 ou 4, **caractérisé en ce que** le milieu colorant est une enzyme qui convertit un substrat en un produit coloré qui est perceptible visuellement et/ou qui est mesurable quantitativement avec un dispositif approprié.

6. Procédé selon l'une quelconque des revendications 1 à 5, **caractérisé en ce que** l'on détermine, pour chaque échantillon, si les anticorps appartiennent à la classe des IgM, des IgG ou aux deux.

7. Procédé selon l'une quelconque des revendications précédentes, **caractérisé en ce que** l'interprétation des bandes de test s'effectue par une comparaison des intensités des signaux des antigènes individuels, dans lequel les antigènes individuels sont référencés sur un « seuil » et dans lequel pour chaque antigène, l'intensité du signal est déterminée, dans lequel l'évaluation suivante est établie :
| **Intensité du signal** | **Évaluation** |
|---|---|
| pas de réaction | - |
| réactivité en dessous de la bande seuil | +/- |
| réactivité au niveau de la bande seuil | + |
| réactivité supérieure à la bande seuil | ++ |
| réactivité nettement supérieure à la bande seuil | +++ |
| réactivité maximale | ++++ |

8. Procédé selon l'une quelconque des revendications précédentes, **caractérisé en ce qu'**il s'agit d'un test ELISA, dans lequel un antigène est placé dans chaque récipient de réaction, ou **en ce qu'**il s'agit d'un dosage sur bandelette et **en ce que** l'intensité de couleur est déterminée quantitativement avec un dispositif de mesure et le rapport de l'intensité des réactions individuelles est déterminé par rapport à la valeur seuil à l'aide d'un dispositif d'évaluation.

9. Trousse de test pour la réalisation d'un procédé selon l'une quelconque des revendications 1 à 8, **caractérisée en ce que** la trousse de test contient plusieurs phases solides spatialement séparées les unes des autres, dans laquelle la trousse de test présente au moins les antigènes suivants :
aa) la « protéine non structurale 1 » d'un premier arbovirus ou une partie immunologiquement réactive de celle-ci présentant au moins 8 acides aminés,
bb) une « protéine d'enveloppe » d'un premier arbovirus ou une partie immunologiquement réactive de celle-ci présentant au moins 8 acides aminés,
cc) une « protéine d'enveloppe » d'un deuxième arbovirus ou une partie immunologiquement réactive de celle-ci présentant au moins 8 acides aminés,
dd) la « protéine non structurale 1 » d'un troisième arbovirus ou une partie immunologiquement réactive de celle-ci présentant au moins 8 acides aminés et
ee) une « protéine d'enveloppe » d'un troisième arbovirus ou une partie immunologiquement réactive de celle-ci présentant au moins 8 acides aminés
ainsi que des réactifs pour le lavage du complexe immun et des réactifs pour la conversion du complexe immun en un signal optiquement perceptible et un sérum prédéfini qui est utilisé comme échantillon seuil, **caractérisée en ce que** le premier arbovirus est un virus de la dengue de sérotype 2, le deuxième arbovirus est un virus du chikungunya, le troisième arbovirus est un virus du zika.

10. Trousse de test selon la revendication 9, **caractérisée en ce que** la trousse de test présente en outre l'antigène suivant :
ff) un peptide présentant au moins 8 acides aminés de la région NS1 d'un quatrième arbovirus, dans laquelle le quatrième arbovirus est un virus de la fièvre jaune.

11. Procédé selon l'une quelconque des revendications 1 à 8 ainsi que trousse de test selon la revendication 9 ou 10, **caractérisés en ce que** la partie immunologiquement réactive des antigènes présente au moins 40 acides aminés.

12. Procédé selon l'une quelconque des revendications 1 à 8 ainsi que trousse de test selon l'une quelconque des revendications 9 à 10, **caractérisés en ce que** la partie immunologiquement réactive des antigènes utilisés comprend au moins 80 % de la longueur totale de l'antigène existant à l'état naturel.

13. Procédé selon l'une quelconque des revendications 1 à 8 ainsi que trousse de test selon l'une quelconque des revendications 9 à 10, **caractérisés en ce que** la partie réactive des antigènes utilisés comprend au moins 95 % de la longueur totale de l'antigène existant à l'état naturel.


### SEQUENZPROTOKOLL

<110> Mikrogen GmbH
<120> Verfahren zur immunologischen Diagnose einer Probe mit einer potentiellen Infektion eines Arbovirus und hierfür geeignete Testkits
<130> PWO00294MIK
<140> EPA 16194496.2
   <141> 2016-10-19
<160> 5
<170> PatentIn version 3.5
<210> 1
   <211> 114
   <212> PRT
   <213> Dengue virus
<400> 1
   Met Tyr Ser Met Cys Thr Gly Lys Phe Lys Val Val Lys Glu Ile Ala
   1               5                   10                  15
   Glu Thr Gln His Gly Thr Ile Val Ile Arg Val Gln Tyr Glu Gly Asp
               20                  25                  30
   Gly Ser Pro Cys Lys Ile Pro Phe Glu Ile Met Asp Leu Glu Lys Arg
           35                  40                  45
   His Val Leu Gly Arg Leu Ile Thr Val Asn Pro Ile Val Thr Glu Lys
       50                  55                  60
   Asp Ser Pro Val Asn Ile Glu Ala Glu Pro Pro Phe Gly Asp Ser Tyr
   65                  70                  75                  80
   Ile Ile Ile Gly Val Glu Pro Gly Gln Leu Lys Leu Asn Trp Phe Lys
                   85                  90                  95
   Lys Gly Leu Glu Val Leu Phe Gln Gly Pro His His His His His His
               100                 105                 110
   His His
<210> 2
   <211> 117
   <212> PRT
   <213> Zika virus
<400> 2
   Met Tyr Ser Leu Cys Thr Ala Ala Phe Thr Phe Thr Lys Ile Pro Ala
   1               5                   10                  15
   Glu Thr Leu His Gly Thr Val Thr Val Glu Val Gln Tyr Ala Gly Thr
               20                  25                  30
   Asp Gly Pro Cys Lys Val Pro Ala Gln Met Ala Val Asp Met Gln Thr
           35                  40                  45
   Leu Thr Pro Val Gly Arg Leu Ile Thr Ala Asn Pro Val Ile Thr Glu
       50                  55                  60
   Ser Thr Glu Asn Ser Lys Met Met Leu Glu Leu Asp Pro Pro Phe Gly
   65                  70                  75                  80
   Asp Ser Tyr Ile Val Ile Gly Val Gly Glu Lys Lys Ile Thr His His
                   85                  90                  95
   Trp His Arg Ser Gly Leu Glu Val Leu Phe Gln Gly Pro His His His
               100                 105                 110
   His His His His His
           115
<210> 3
   <211> 32
   <212> PRT
   <213> Dengue virus
<400> 3
   Lys Arg Ser Leu Arg Pro Gln Pro Thr Glu Leu Lys Tyr Ser Trp Lys
   1               5                   10                  15
   Thr Trp Gly Lys Ala Lys Met Leu Ser Thr Glu Ser His Asn Gln Thr
               20                  25                  30
<210> 4
   <211> 15
   <212> PRT
   <213> Dengue virus
<400> 4
   Pro Trp His Leu Gly Lys Leu Glu Met Asp Phe Asp Phe Cys Asp
   1               5                   10                  15
<210> 5
   <211> 24
   <212> PRT
   <213> Dengue virus
<400> 5
   Asp Ser Gly Cys Val Val Ser Trp Lys Asn Lys Glu Leu Lys Cys Gly
   1               5                   10                  15
   Ser Gly Ile Phe Ile Thr Asp Asn
               20